# EUROPEAN PATENT SPECIFICATION

(11) **EP 2 866 210 B1**
(45) Date of publication and mention of the grant of the patent: **22.07.2020**
(21) Application number: 13189904.9
(22) Date of filing: 23.10.2013
(51) Int. Cl.: G07F 17/00

(54) **High efficiency automated pharmaceutical dispenser**
Hocheffiziente automatisierte pharmazeutische Spender
Distributeur automatisé de produits pharmaceutiques à haut rendement

(43) Date of publication of application: 29.04.2015
(73) Proprietor: Generation Unlimited, LLC, Huntington Beach, CA 92547 (US)
(72) Inventor: Brown, Arthur E, Mission Viejo, CA 92692 (US)
(74) Representative: Appleyard Lees IP LLP

(56) References cited:
- WO-A1-2010/065845
- US-A1- 2005 240 305
- US-A1- 2012 145 585
- US-B2- 8 036 773

## Description

### FIELD OF INVENTION

This invention relates to automated pharmaceutical dispenser devices such as those that dispense a plurality of different drugs with varying doses used in hospitals, pharmacies and home health care facilities.

### BACKGROUND

The dispensing of pharmaceuticals in hospitals, pharmacies, home healthcare, assisted living and similar facilities is a critical aspect of patient care. Pharmaceuticals are manufactured by numerous drug companies, most using different types of packaging, or packaging that is not uniform in size, drug quantity, labeling, or dosage. These packages can be syringes, ampules, vials, oral suspensions, tubes, jars, blister packs in single or multiple dose sheets, and many bottles of various sizes and shapes. The lack of standardization results in confusion for medical professionals regarding the delivery proper dose and medication, and it is known to result in a large number of adverse drug reactions caused by errors in the stocking, storage and delivery of prescribed medication.

Historically, in a large multi-patient environment, like hospitals that can have hundreds of beds, prescriptions are written by doctors; the prescription is physically or electronically presented to a hospital pharmacy; the pharmacy picks and packs the medicine for physical transfer to a cart or tray for transfer to nurses for delivery to and consumption by patients in their rooms. Nurses are usually responsible for multiple patients located in different rooms or locations within the hospital. Each step in the delivery chain opens opportunities for mistakes in giving patients an improper dosage or improper medication. In reading poor hand writing or inverting numbers on a script, pharmacists may accidently provide the wrong dosage or drug for delivery to a patient. Errors may also occur during transport to the patient's room or during the administration of the drug by nurses. These errors result in many serious or fatal adverse drug reactions every year and cost our health care system many billions in excess costs annually.

Attempts to improve existing packaging, storage, script writing and delivery systems and methods have been made. Systems are known with automated computerized script writing, cross referenced against electronic digital patient medical record, automated storage and dispensing. U.S. Pat. No. 6,757,898 discloses an electronic tracking and patient cross checking systems that is a significant improvement over manual systems. Doctors can now place scripts at a patient's bedside electronically through tablet computer and smart phones that are networked to interface directly with patient records and pharmacies. RFID and barcode systems are known that provide significant improvements in identifying and tracking drug type and dosage as the medication flows from script to patient. Further advances have been made with inventory management, tracking and control, reordering and stock adjustment systems. The security of inventory has also been improved by providing user authorization and authentication with delivery confirmation systems that allow for only dispensing drugs to authorized individuals and tracking the delivery of the dose until confirmation of delivery is provided.

Some attempts have been made to establish standardization in bar coding. 21 CFR 201.25 sets out guidelines for the pharmaceutical industry with respect to bar code formats and requirement for certain types of data. However, even with these advancements, there continue to be deficiency with these systems. Because there are no established standards for packaging, handling, tracking, dispensing and delivery of drugs in institutional environments, there remain significant inefficiencies, errors and limitations with existing designs. There is also a significant lack of standardization in the nature and structure of data that is captured and used in managing these functions. Automated dispensing machines have a number of limitations because they are generally designed to handle a variety of package designs or they require a significant amount of manual effort to stock or restock. Current state of the art automated dispensers, in order to handle a variety of medications, also require the manual preparation of individual unit doses of medication so that automated systems can accommodate the package for automated processing. Unit doses must be physically separated and placed in individual bin locations or canisters within the automated dispenser.

There are also limitations with respect to inventory monitoring and control of inventory in current pharmaceutical dispensers. In existing systems multiple individuals may have a key or access to secured areas or access point where medication is stored and inventoried. This leaves inventory vulnerable to unauthorized removal or theft.

Additionally, many of the known systems are very inefficient in both the unit dose package storage density and in the process of stocking and restocking of pharmaceuticals. In one known system, the McKesson Automation, Inc. system disclosed in U.S. Patent No. 8,036,773 is designed to hold unit dose packages of various sizes. However, the McKesson system requires that each unit dose package be individualized or separated from multipack packages and that each separated unit dose package be place in individual carriers in a horizontal plane. The separation of the individual unit dose package is a manual process and requires a significant amount of physical labor to separate and load individual unit dose packages or to otherwise manipulate the unit dose packages to allow accommodation of different package sizes by the system. Alternatively, the user must purchase a standalone separating machine for the purpose of separating unit dose packages, which adds significant cost.

Because the system disclosed in U.S. Patent 8,036,773 requires that each individual unit dose package be loaded into a carrier and then multiple carriers are stacked into a storage apparatus, there is a significant amount of unutilized space within the system and the unit dose package density is extremely low, requiring constant manual stocking as described above. Each time the system is stocked there is opportunity for error, and cost is added through manual processing. The loading or stocking procedure is just as lengthy and requires as much operator time as does the dispensing.

U.S. Patent No. 8,090,472 issued to Schifman et al discloses an automated medication dispensing apparatus. This dispenser is similar to the dispenser disclosed in U.S. Patent 8,036,773 in that it uses multiple pharmaceutical storage bins with multiple compartments for holding unit dose packages. The storage bins are stacked and each has an assigned location within a cabinet or enclosure. A robotic arm selects a pharmaceutical by selecting the proper bin location and moving the robotic arm to the bin location to extract the pharmaceutical stored at that location. The same limitations apply, in that there is low storage density, high manual processing and associated increase in error rates. The Schifman dispenser does improve security by including a camera for capturing still or video images of users accessing the apparatus.

Pharmaceutical dispensers have also improved by allowing digital communication with computer networks. Many healthcare facilities use integrated medical records management software to assist in patient care and to efficiently make available to clinicians patient information. Doctors can enter prescriptions into mobile computer devices such as tablets and smart phones. These wireless devices can be networked to centralized servers or cloud based databases that can interface with automated pharmaceutical dispensing systems. U.S. Patent 8,090,471 discloses at a conceptual level such a system. These software applications have significantly improved the efficiency of the overall drug delivery process in healthcare facilities by reducing or eliminating mistake in script writing, patient identification. Software is also known for assisting in the management of inventory and access authorization in the automated pharmaceutical dispenser systems. However, these systems cannot improve efficiencies based on the lack of standardization or the limitations of the underlying automated dispenser design.

### Personal Automated Dispensers

As the causes of mortality have shifted over the past one hundred years from acute infectious disease to chronic disease such as cardiovascular disease, cancer, diabetes and other age related diseases, pharmaceutical and biotech companies have developed a plethora of treatments that can be self-administered by patients without hospitalization and only minimal physician oversight. Patients with chronic ailments may often have multiple drugs that are taken at various times during the day. As lifespans increase and populations age, the challenge of managing medication schedules becomes more difficult and for some require assistance. Failure to maintain ones medication schedule can create serious medical problems for the patient. Additionally, some patients may have multiple prescriptions and can be confused about which drug relates to the appropriate schedule of administration, resulting in taking drug A on schedule intended for drug B. Additional problems exist with these patients simply failing to take their medication.

A number of personal automated medical dispensing devices are known. E-pill, LLC (www.epill.com/dispenser.html) manufactures a full line of personal dispensers having many of the features of larger systems but scaled to individual users. Many systems are microcontroller based and can have sophisticated user interfaces that allow users to set a number of system functions and features. A key feature of personal dispensers is a medication administration scheduling feature that provides notification to the user or healthcare providers of the time to take medication. Notification can be done via audible indicator, light flash, or wireless communication to a third person when medication is not removed from the dispenser at the appointed time. Although personal automated medical dispensers have improved, many of the same limitations existing with automated dispensers used in institutional venues carry over to personal automated medication dispensers, with some additional limitations. Much of the stocking procedure for personal automated dispensers is carried out manually, resulting in a system that is prone to error. Because of the smaller size of personal drug dispensers, restocking is required more often than larger automated systems, providing for even more opportunity for error. Additionally, many patients may be impaired either physically or cognitively and thus are incapable of properly stocking the dispenser and requiring assistance from a medical professional or family member for restocking. There are similar applications in the retail pharmacy, so called lights out order fulfillment and mail order facilities.

Patent publication WO 2010/065845 A1 describes a pharmaceutical dispensing system and associated method. Patent publication US 2012/145585 A1 describes packaging systems and methods.

### SUMMARY OF THE INVENTION

In view of the foregoing background, the present invention overcomes the limitations of the prior art by providing for a high efficiency automated pharmaceutical packaging method and dispensing systems for hospital, pharmacy, residential and home healthcare facilities.

According to the present invention there is provided an apparatus and method as set forth in the appended claims. Other features of the invention will be apparent from the dependent claims, and the description which follows.

In another aspect, there is provided a pharmaceutical dispensing apparatus as set out in the accompanying claims.

One or more features of one or more aspects may be replaced with and/or combined with one or more features of one or more other aspects, unless clear to the skilled person that such replacement and/or combination would be technically mutually exclusive.

### BRIEF DESCRIPTION OF THE DRAWINGS

Embodiments of the present disclosure will be more readily understood by reference to the following figures, in which like reference numbers and designations indicate like elements.
FIGURE 1A is a profile view of one embodiment of one aspect of the invention showing a section of the ribbon or tape with segments and cavities with the removable covering.
FIGURE 1B is a top view of the tape with segments and cavities and removable covering.
FIGURE 1C is a profile view of a single unit dose after being dispensed and removed from the ribbon.
FIGURE 2A is a front view schematic representation of a length of continuous ribbon containing unit doses on a minimal carrier comprised of a central core and sufficient side supports to keep the tape manageable when handled outside of a dispenser or other restraint.
FIGURE 2B is a front view schematic representation of a length of continuous ribbon containing unit doses on a core supported and protected by circular sides.
FIGURE 2C is a front view schematic representation of a carrier which may provide a stand-alone dispensing method, a shipping container, or a structurally independent and uniform cassette that is inserted into a dispenser for automated dispensing.
FIGURE 2D is a schematic representation of a length of unit dose ribbon of comparable quantity to a blister pack sheet of unit doses. Also shown is an embodiment of a tube into which a length of unit dose ribbon can be placed.
FIGURE 2E is a schematic view of the end of a tube with unit dose packages inserted and mechanical means of both restraining and permitting the advancement of a unit dose out of the end of the tube.
FIGURE 3A is a schematic representation of a portion of a dispenser showing 2 reels of unit dose medications being presented at individual locations where the dispensing locations are closer together than the respective dimensions of each reel; also shown is an embodiment of presentation heads with a single unit dose presented according to the present teachings.
FIGURE 3B is a schematic representation of a portion of a dispenser with tubes being used rather than reels. The tubes are shown at an angle to the presentation head to demonstrate the advantage of the flexible ribbon packaging and how the density of the presentation heads is independent of the density of the storage media.
FIGURE 4 is a front view representation of a unit dose package at the presentation point being constrained by a pair of front stops.
FIGURE 4B is a side view of a unit dose package at the presentation point with the upper constraint lifted to allow the unit dose package to be pulled forward by the dispensing head, and a modified embodiment showing the lower presentation platform tilted down on pivot to allow increased access to the unit dose package for dispensing and electronic reading of indicia.
FIGURE 4C is a schematic view of a presentation head without a unit dose package present.
FIGURE 4D is a side view of the mechanical restraint of a presentation head showing the forward restraints.
FIGURE 5 is a schematic view of the back of a cabinet typical of a hospital pharmacy application where appropriate lengths of UPD ribbons are contained in tubes, or loaded directly into slots.
FIGURE 6 represents a profile view of a mechanical system of UPD ribbons rolled onto reels being stored and dispensed in a high density system.
FIGURE 7 represents a view of a portion of the back of the cabinet of figure 5 with lengths of UDP ribbons either in tubes or independent of tubes in position to be dispensed.
FIGURE 8: Represents one embodiment of a complete system with the various components of the system.
FIGURE 9 is one embodiment of the dispensing head aspect of the present invention of a dispensing head.
FIGURE 10 depicts a front view of an embodiment of a home or personal dispenser.
FIGURE 11 depicts a front, top down and profile view of a single cassette for a home dispenser.

### DETAILED DESCRIPTION OF THE PREFERRED EMBODIMENTS

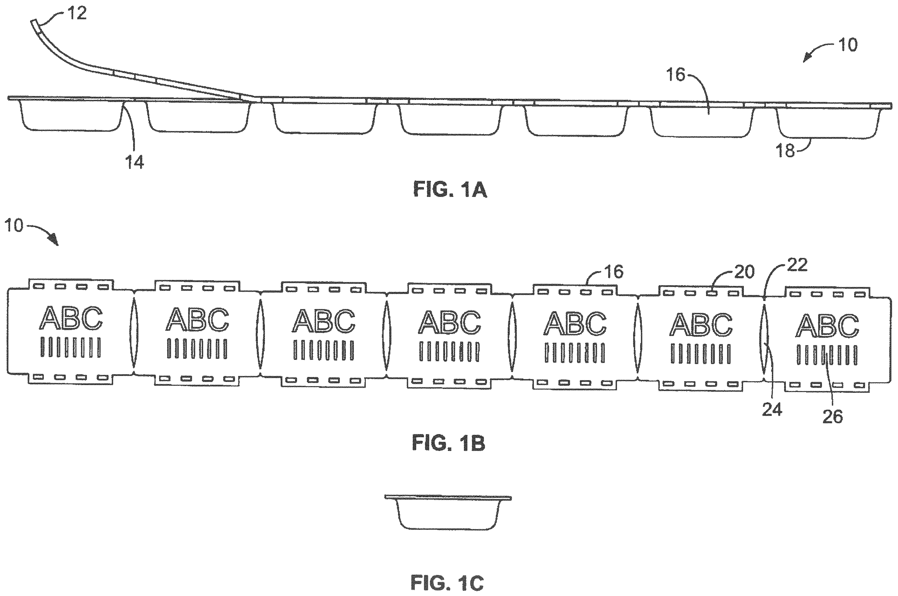

The present invention provides for a high efficiency automated pharmaceutical packaging and dispensing system for hospital, pharmacy, residential and home healthcare facilities. The present invention will now be described more fully with reference to the accompanying drawings, which shows the preferred embodiments of the invention. This invention may, however, be embodied in many different forms and should not be construed as limited to the illustrated embodiments disclosed. Rather, these embodiments are provided so that this disclosure will be thorough and complete, and will fully convey the scope of the invention to those skilled in the art. Like numbers refer to like elements throughout. The preferred embodiments of the current invention and methods will now be described in detail, with reference made to FIGURES 1-11. FIGURES 1-11. Referring now to the drawings, where the showings are for purposes of illustrating the preferred embodiments of the invention-only and not for purposes of limiting the same.

FIG 1A is a side profile representation of the high-density packaging ribbon segment , or unit dose package (UDP) 10 for pharmaceutical unit doses processed in a high efficiency automated pharmaceutical dispensing systems. The ribbon could also be a band or tape suitable as a packaging material. The ribbon of the preferred embodiment is linear; however, it is contemplated that the ribbon may be multidimensional and composed of multiple columns and row. Pharmaceuticals are packaged in a ribbon of packaging material of a desirable width. The ribbon can be of essentially any width and length depending on the dispensing application and the pharmaceutical contained within the ribbon. The ribbon 10 is comprised of a flat layer 12 and a cavity layer 14 sealed together along a single length of all sided of the cavity, sufficient to keep the medication contain within the cavity or if preferred air tight, using any appropriate means such as a strong adhesive. The cavity layer may simply be a shrink wrap material that provides sufficient space or volume for the unit dose of the medication being packaged in this specific ribbon. The ribbon comprises a plurality of individual segments 16, each with a cavity 18 for holding individual unit doses of a pharmaceutical or individual container such as a bottle, vile or syringe containing such pharmaceutical. Each ribbon segment is sequentially positioned on the ribbon so that there is only one dose or cavity per segment within the width of the ribbon package for each segment unit length, but multiple essentially identical segment lengths sequentially and uniformly spaced on the ribbon. The individual segments may be completely sealed or have intentional holes such as in the bottom of the cell to allow for pressure variation, circulation of air, or to assist in the dispensing process by allowing access to the pill or to advance the ribbon to the next segment. It is also contemplated that for ointments or a topical the ribbon may be comprised of a series of individual pouches, similar to ketchup pouches which are strung on a single ribbon length. To use the same ribbon and reel strategy for personal dispensers, it is possible to have a master dispenser feed medication into a second ribbon and reel container directly at the time of prescription filling so that a custom ribbon is created with serially sequential dosages of various drugs are placed in a length of ribbon for dispensing at a home or care facility by a device that is intended for a single patient.

FIG 1B is a top down view of the flat layer 12 of the ribbon 10. Each ribbon segment 16 may have sprocket holes 20 or notches 22 or other similar physical features such as embossments on either or both linear edges to that are used as register points and that allow the ribbon to be pulled or drawn from a reel to advance each ribbon segment 16 through the system. Such means may also be used to move or advance the ribbon products through manufacturing or filling and sealing and the several dispensing operations. These notches 22 can be located symmetrically at some distance from the leading edge of the segment so that either end of the ribbon can be the leading end or the notches can be offset or tapered so that only one end of the ribbon can be the leading end and the other the trailing end, the ribbon advancing in only one direction. The ribbon 10 may include perforations 24 or cut and removed space between each segment 16 that extend the width of the ribbon and allow for easy separation of segments.

On each segment 16 of the ribbon 10 information 26 is included that may be human or machine readable. The information 26 can represent any information relevant to the particular pharmaceutical, such as name, dose, manufacturer date and lot code, or unit identification of the individual segment on the ribbon reel location. Each side of individual ribbon sections may contain encoded data indicative of relevant information. The data can be essentially any type of data and it can be encoded in a variety of know means, including single or multidimensional bar code. The data can be read as the ribbon segment passes over a reader to compare against the script to assure the proper medication is provided. FIG 1C is a front view of an individual ribbon segment.

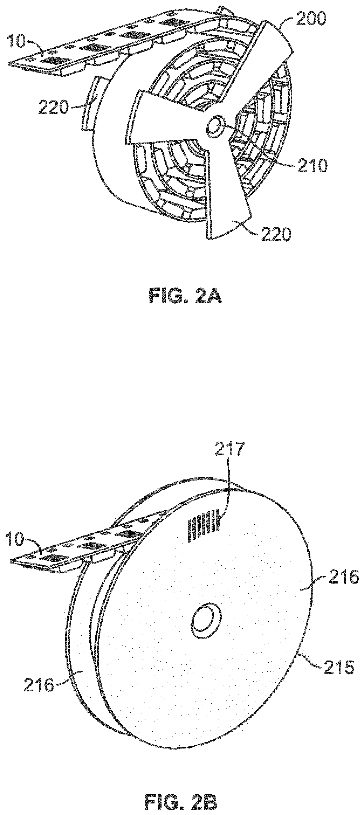

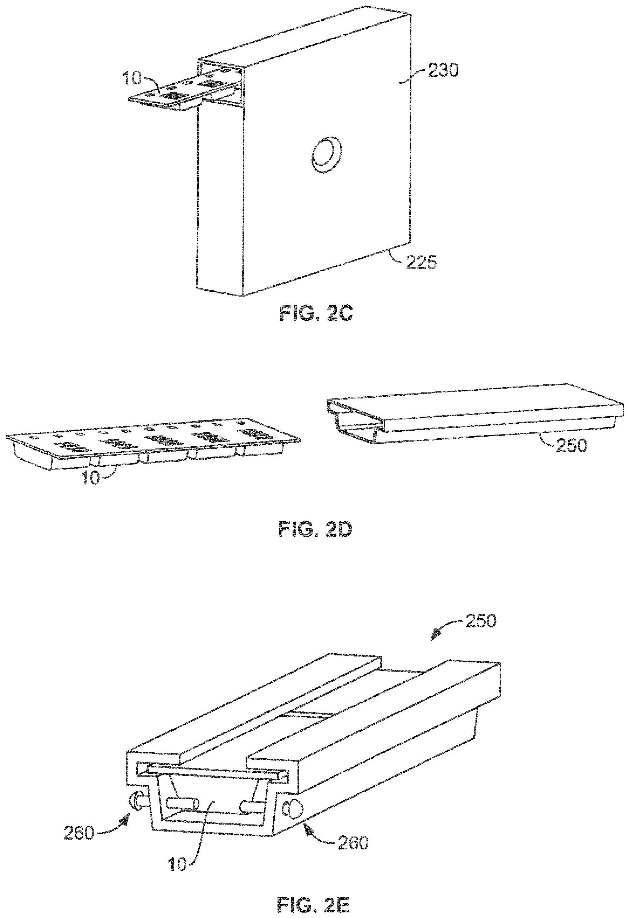

The ribbon with individual unit doses packaged within each ribbon segments is further packaged for use in the system. The ribbon packaging allows for the automation and uniform transport, tracking, storage and dispensing in a highly efficient manner. Now with reference to FIG 2A, the ribbon 10 is wound about a reel 200 with a center core 210 that allows for the placement of the reel on a sprocket. The reel 200 that may incorporate generally circular side supports 220 of such size and configuration as to create an overall package with integrity onto which a desirable ribbon length of prepackaged individual unit doses of drugs can be wound. FIG 2B is an alternative embodiment showing a ribbon 10 wound about a reel 215 with full circular side supports 216. The benefit of the full side support is the greater strength of the overall package and the added surface area for display of greater quantities of printed information 217. FIG 2C shows a ribbon 10 wound about a reel (not shown) contained in cassette or cartridge 225 for easy warehousing, transportation, storage and placement within the dispensing system. The cartridge 225 is preferable in hospital and pharmaceutical applications to increase storage capacity, prevent contamination, and to make restocking of the system more efficient. The cartridge 225 is formed from ridged support panels 230 made from any suitable material and that fully enclose the reel. It is also contemplated that standardized packaging may also be a container or box into which a fan-folded ribbon may be placed.

FIG 2D presents another alternative embodiment of the ribbon packaging 10 that is preferable in smaller applications such as a home health care environment or for personal dispensers, allowing for smaller quantities and thus greater varieties, (more SKU numbers). Each length of ribbon 10 would approximate the number of UDP's on a blister card, and thus the minimum order quantity (MOQ) that a hospital would receive in an order. One of the benefits of this design is the dramatic reduction, if not elimination, of an inventory area outside of the dispenser. With the enhanced density and reduction of manual labor required to 'singulate' UPD's from their parent blister cards, as well as not having to consume time filling loading trays from which a robot picks up UPD's to place into a dispenser, incoming pharmaceutical inventories can be placed directly into the invention in any available location.

Now with reference to FIG 2D and 2E, each ribbon 10 is cut into smaller strip lengths of a set number of unit dose ribbon segments 16 that may be fed into a tube 250 or similar conduit restraint system. The tube 250 shown in FIG 2D and FIG 2E conforms to the general shape of the ribbon's leading edge front profile, which allows for convenient insertion into the dispenser. FIG 2E shows the ribbon 10 inserted packaged in the tube 250. The tube 250 may contain ribbon stop restraints 260, which are engaged by the dispenser to allow the ribbon to advance and prevent the ribbon 10 from slipping from the tube 250.

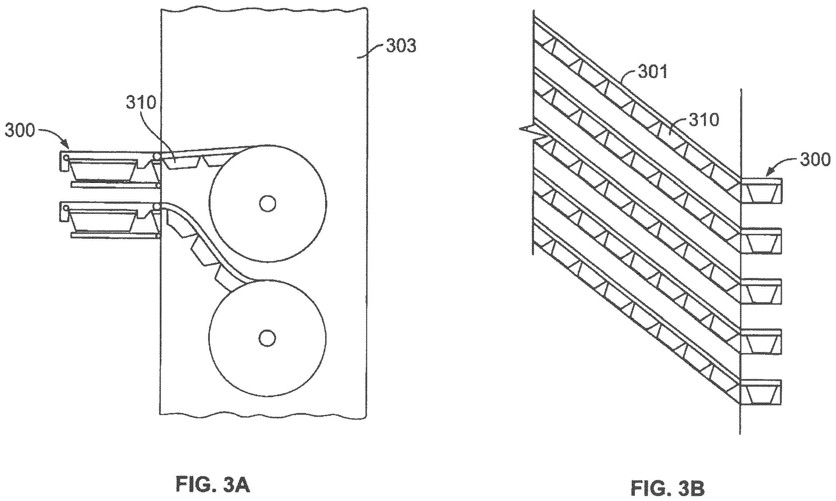

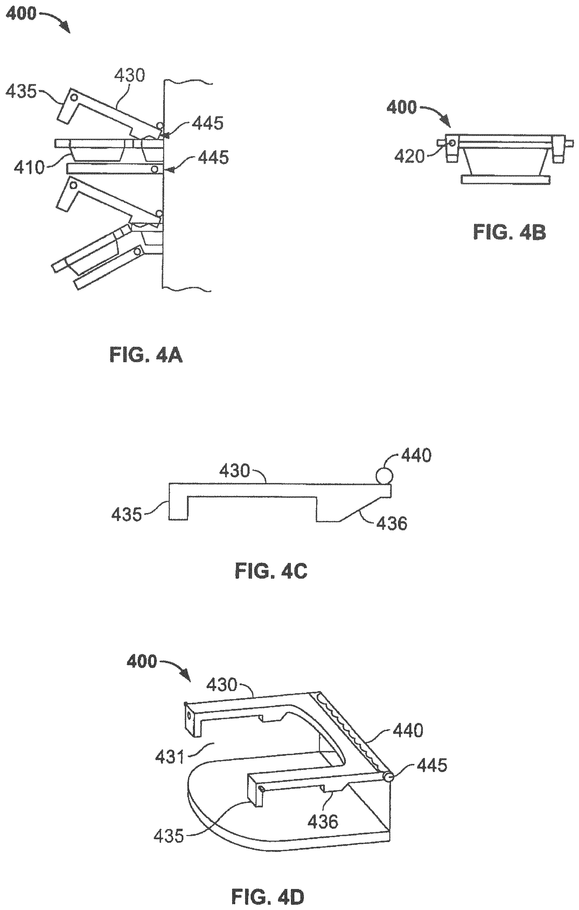

Referring now to FIGS 3A, and 3B, a presentation head 300 may be incorporated into the conduit 301, reel, cassette or cartridge 303 for serially presenting or separating each reel ribbon segment 310. One skilled in the art will appreciate that a number of alternative designs can be engineered for achieving the same objective of storing, advancing and presenting the ribbon. FIGS 4A, 4B, 4C, and 4D show different views of the presentation head. The presentation head 400 will feed or position each ribbon segment 410 into the automated dispensing device so that the each ribbon segment and its contents would be presented for dispensing in a way where after the dispensing a first ribbon segment, the next ribbon segment will be advanced to the dispensing position and available for a dispensing head. The dispensing head 400 may incorporate an optical target or alignment sensor 420 at its leading end for ensuring the proper alignment of a picking head (not show).

The upper arm portion 430 of the dispensing head preferably has a central open space 431 that allows a reader to have visibility access of the ribbon as it proceeds through the presenting head into the dispenser head. The presentation head 400 may be associated with a reader (not shown) for reading the encoded data on each ribbon segment which may be identified with human and machine readable elements such that a dispensing head can be directly and uniquely associated with a specific reel or cartridge so that the head's identity data defines the pharmaceutical that is dispensed. The reader reads data from the ribbon surface and communicates this data to the system.

The upper arm 430 preferably hinged at the rear and contains a spring 440 or other mechanically created load at a hinged location 445 to keep the upper arm 430 in a closed position unless the ribbon segment is pulled through the head. A front register 435 and a back registers 436 will limit the advancement or prevent backward movement of the ribbon as it is pull through the dispensing head by closing on the register notch located between each ribbon segment. As the ribbon is pulled through the dispensing head the upper arm 430 raises until a register notch is reach and the spring 440 tension forces the upper arm 430 to close at the register notch.

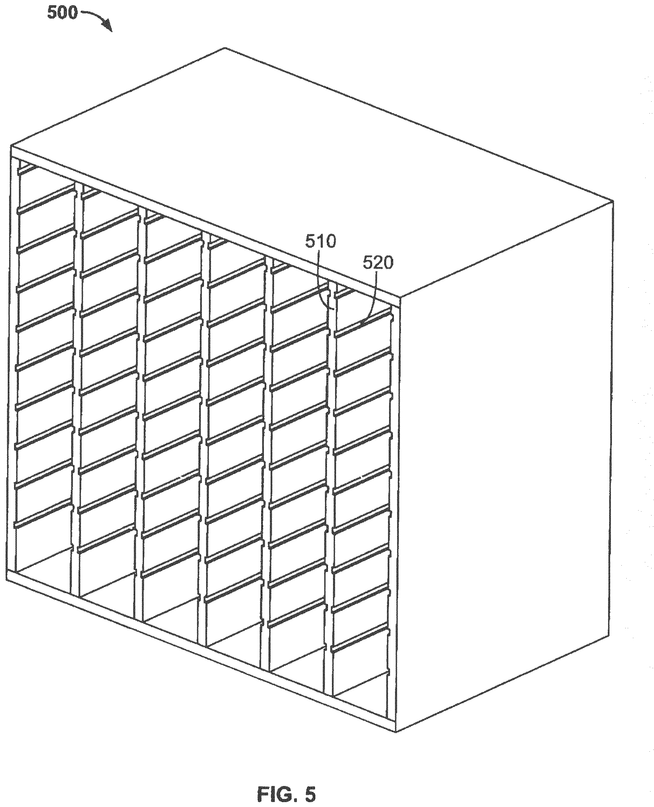

Now referring to FIG 5, the system includes a dispensing cabinet 500. The cabinet can be of any shape and size, but is preferably structured to accommodate the particular application and environment where the system is used. In one embodiment shown is FIG 5, the cabinet 500 is a seventy two inch by thirty six inch box enclosed on the top, bottom and sides. The cabinet has a series of dividers 510 running vertically and spaced 1.5 inches apart. Each divider 510 has a plurality of grooves 520 on each side of the divider and spaced 1.5 inches. This configuration provides for 1,152 slotted locations. If each slot will have a location address and is loaded with ribbons containing twenty four segments the total contents of the cabinet will be 27,648. However, if a reel containing a ribbon with two hundred segments is used the total content can be increased to 230,400 unit doses. Thus the storage density advantage of the ribbon and reel configuration is apparent.

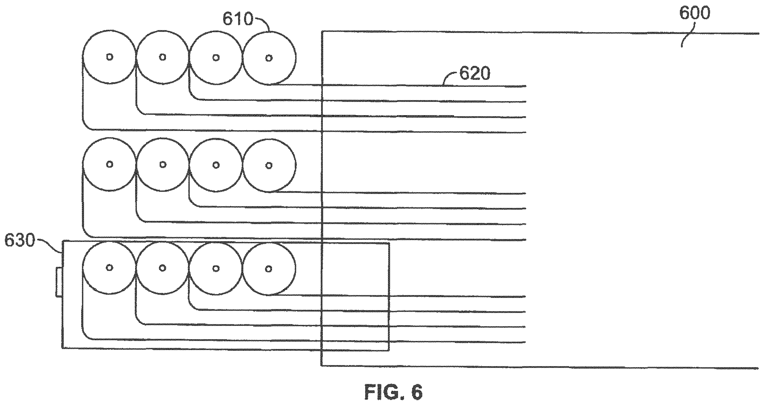

FIG 6 demonstrates one configuration for installing multiple reels 610 into the cabinet 600. Each reel 610 is placed in a slot conforming to the size of the reel. Multiple reel slots are structured in a drawer 630 that can be pulled open to replace a reel. The ribbon of each reel is fed through feed slots 620 within the cabinet.

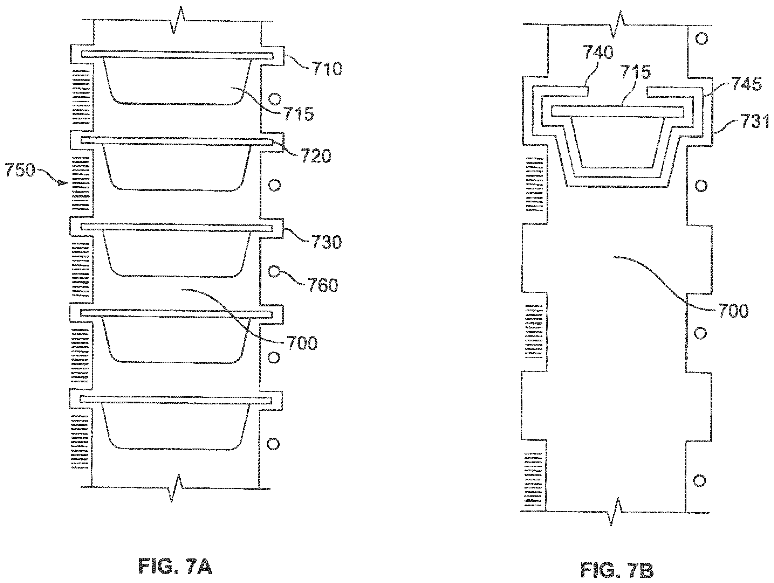

FIG 7 shows the slots configuration of one column within the cabinet 700. Referring to 7A, in an embodiment where ribbons 715 are fed directly though the vertical slot 710. Lateral extensions 720 fit into grooves 730 of the slots. Each slot location is provided an address or coordinate that allows for identification of the location. The address data can be represented as bar code or other data associated 750 with the slot location or associated with a sensor that is triggered when inserting a ribbon. Additionally, a user may input other data such as drug type, dose, quantity, etc. An indicator light 760 is also provided at each slot location, which is illuminated when restocking.

Now, referring to 7B, in an embodiment where a conduit or tube 740 holds the ribbon 715, the conduit is formed to include lateral extensions 745 that fit into grooves 731 of the slots. A clip or other means such as a door, pin, slide is used for preventing the conduit from slipping from the slot.

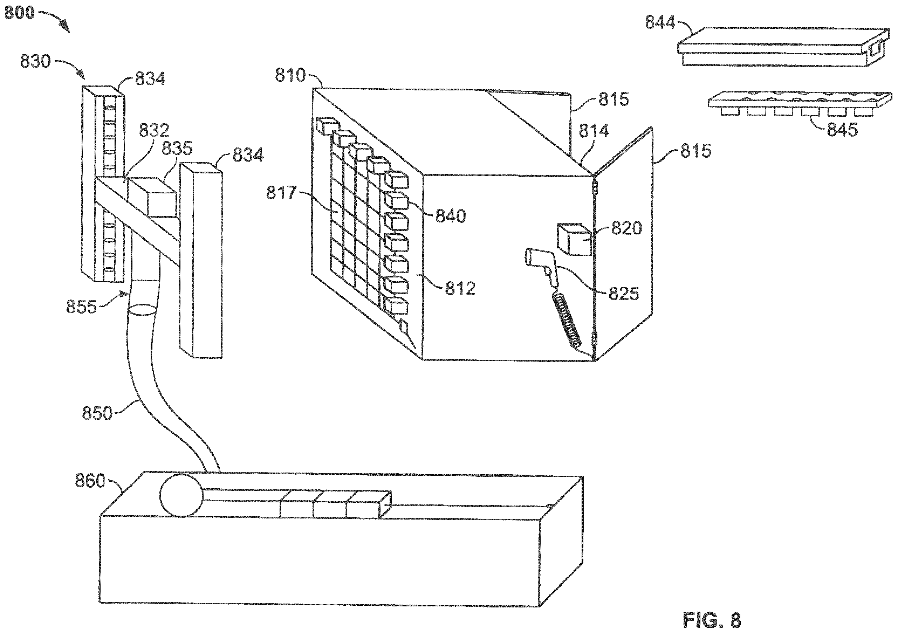

Now referring to FIG 8, illustrated is the overall configuration of the preferred embodiment of the system 800. The system is comprised of storage cabinet 810 having a dispensing face 812 and an inventory loading face 814. Doors 815 are provided for securing inventory internal to the cabinet and for gaining access to a plurality of conduit slots 817 running the length through the cabinet 810. A plurality of presentation heads 840 extend from the slots 817 on the dispensing face side 812 of the cabinet. For illustration purpose only, not every slot 817 of FIG 8 includes a presentation head.

The cabinet will have a user interface 820, which one of skill in the art will appreciate could include many conventional known types of interfaces and may include a keyboard, display, wired or wireless communications interface with other devices. In the preferred embodiment the user interface 820 is microcontroller based and controlled by a software application. The user interface 820 allows users to access the various functions and reports of the system. Additionally, the user interface 820 may be connected to a modem or other wired or wireless communications interface (not show) that will provide communications with a computer network or the Internet (also not shown) and will allow for remote access to, data exchange with and control of the system.

The system 800 includes a data reader 825. In the preferred embodiment the reader 825 is a single or multi-dimensional bar code reader that allows users to scan data from individual conduits 844 packaged with ribbons 845 of unit doses prior to insertion into the cabinet slot 817. The data reader 825 can also be used to read data on individual unit dose packages or at each individual slot location 817. By reading data from the conduit 844 and slot location 817 at the time of stocking inventory into the system the system can track the location of pharmaceuticals of various doses and verify and cross check against patient data or drug interaction data when filling a prescription to ensure there are no errors in drug type or dose. The captured data can also be used to generate a large variety of reports, for inventory management and for system access monitoring.

A dispensing head support frame 830 is interfaced with the cabinet 810 and provides a rigid structure for moving the dispensing head 835 in the X and Y coordinates. The dispensing head support frame 830 includes two upright beams 834 and a cross beam 832, which adds support and provides for a mounting location for the dispensing head 835. The cross beam 832 can be raised and lowered on the Y axis using a mechanical motor means within the upright supports such as a motor driving a belt, drive shaft, linkage system or similar system. The dispensing head 835 can be moved along the X axis using a similar means within the cross beam 832. There are many know means for mechanically moving a load along the X and Y axis. It will be appreciated by one skilled in the art that any of these means can be used to move the dispensing head along the X and Y axis.

When the dispensing head support frame 830 is mounted to the cabinet 810, the dispensing head 835 is movable along the X and Y axes and as it moves from one slot location to the next will interface with the presentation heads 840 located at a plurality of slot location within the storage cabinet 810. The dispensing head 835 will be mapped to the proper slot location based on a grid address system and software that is loaded into a system microcontroller within the user interface, ensuring that proper location is located and unit dose packages are picked. As the system accepts a prescription from authorized users, which can be digitally communicated to the system via linked computer network. The system can cross check against patient records to verify that the unit dose is appropriate for the particular patient's physical data and condition. The system also can use a look up table to make sure there will be no adverse drug interactions based on the patient's current prescriptions.

855 shows a temporary collection device for collecting all the doses required to fill a specific prescription for a single patient. This allows the dispensing head 835 to travel to all required drug locations in the system to dispense the required medications for a single patient before returning to a home or discharge position. 850 is a conduit for receiving the doses from the collection tube 855 and transferring them to a distribution sorting device 860 where each patient's completed prescription is placed in a unique container where a printer print a label and the container will be transported to the patient for administering.

Briefly described, this process includes the picking head 835 being moved to each presentation head 840 required and picking a UPD for each medication required. These UPD's are held in the temporary collection device 855 which is attached to 835 as it moves until the picking process for a single patient is completed. The dispensing head 835 then moves to a position approximate to 850 into which 855 transfers the UPD's to complete the patient prescription. 860 then advances a new pocket opening which is labeled appropriately and into which the UPD's are placed. The pocket is then unsealed. In a hospital scenario, the dispenser is programmed to pick the medications according to the delivery order in which they will be administered. By creating a continuous strip or bandolier of labeled and sealed pouches connected and perforated between in the order in which they will be distributed, accuracy, security and savings of space is achieved.

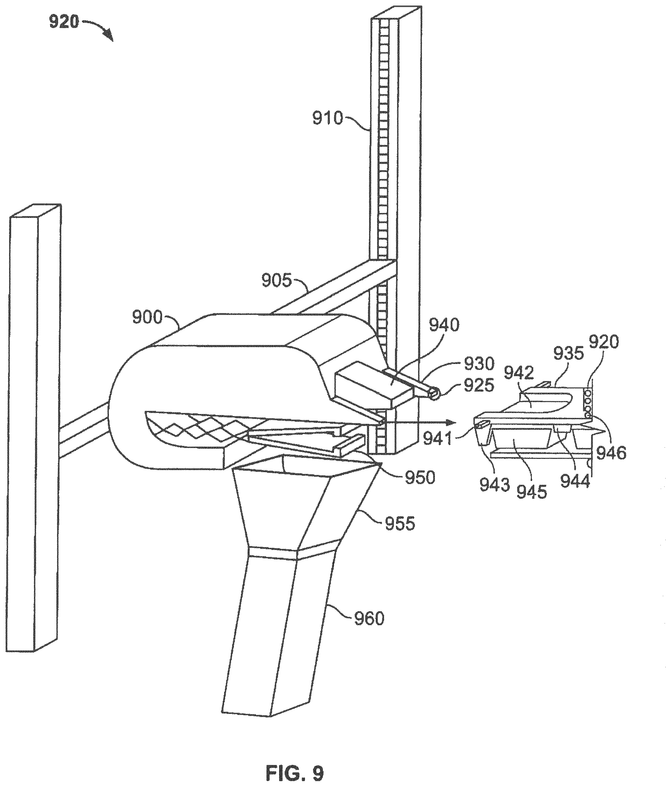

Referring now to FIG 9, the dispensing head 900 is mounted to the dispensing head support frame cross beam 905. Head 900 is moved in the Y direction on upright beams 910 and the X direction on cross beam 905. Once in the proper XY position to access a presentation head according to the prescription, the dispensing head slides into position in the Z direction with the presentation head 920 and an optical sensor 925 detecting a mark or target on the presentation head 920 to allow for proper alignment of the presentation head 920 and the dispensing head 900. A presentation head opener 930 having a wedged shape slides under the opening pins 941 on upper arm portions 935 of the presentation head and lifts the upper arm against the spring tension at the hinge as the dispenser head move forward. A reader 940 moves over the open portion of the upper arm and scans the data 942 on the ribbon segment 945 made available for dispensing, confirming the type of drug, dose, segment number, and other relevant information. A cutter 950 grabs, extracts, cuts and separates the ribbon segment 945, which drops into a collector 955 having an attached chute 960. A front register 943 prevents multiple ribbon segments from advancing and a back register 944 prevents the ribbon from retracting. As the dispensing head 900 retreats from the presentation head 920 the upper arm 935 will move to the closed position as a result of tension caused by the hinge spring 946.

Again referring to FIG 8, once the ribbon segment has been separated from the ribbon it falls through a chute 850 attached to the dispensing head collector 855 to a packaging table 860 where the prescription is prepared for delivery to the patients. Within the packaging table 860 will be a bagger for placing individual ribbon segment unit doses for a specified prescription. The bagger may be on a roll, each bag drawn for each prescription. A printer a bar code label and seals it to the bag to properly identify the contents and associated with the proper prescription. A conveyor delivers the packaged script to a collection area.

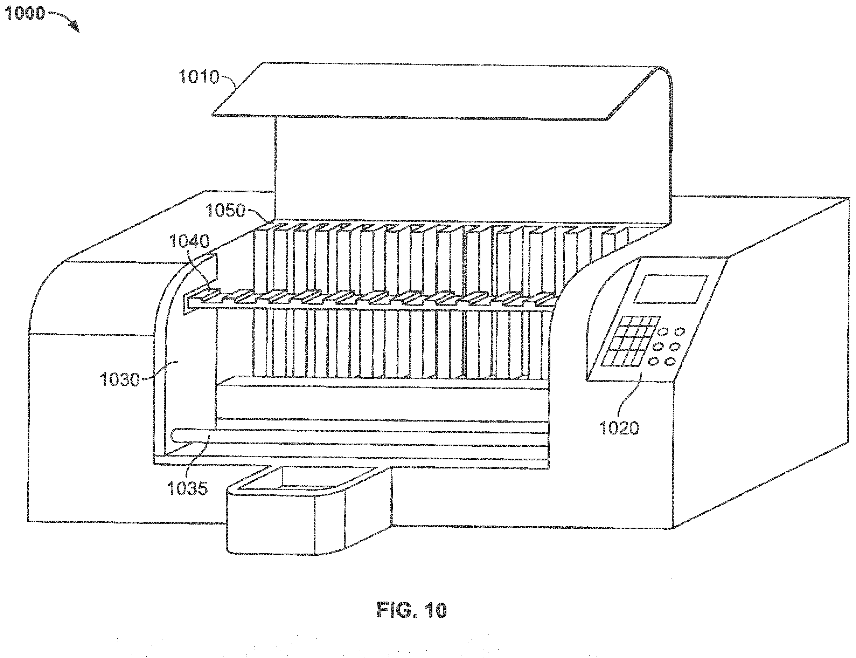

It will be understood by those skilled in the art that the system may be configurable with a variety of different such cabinet types, pick and pack mechanisms and packaging processes. For example a tower or column with multiple bin locations around the circumference of the tower and multiple stacked layers rotating on a carousel for easy presentation of the presentation head to a picking head. Another configuration may have multiple towers surrounding a single dispensing head. Yet another configuration may be a personal and small venue application. FIG 10 shows such a configuration.

With reference to FIG 10, an embodiment for a personal pharmaceutical dispenser 1000 is provided. The personal configuration has the same components of the hospital version, including the door 1010, a user interface 1020, a dispensing head 1030, presentation heads 1040, and medication slot positions 1050. The process is essentially the same as in the larger hospital application without the requirement of any Y movement. The dispensing head 1030 moves by a drive shaft 1035 and aligns with the presentation head 1040 for extracting, cutting and separating the ribbon segment unit doses.

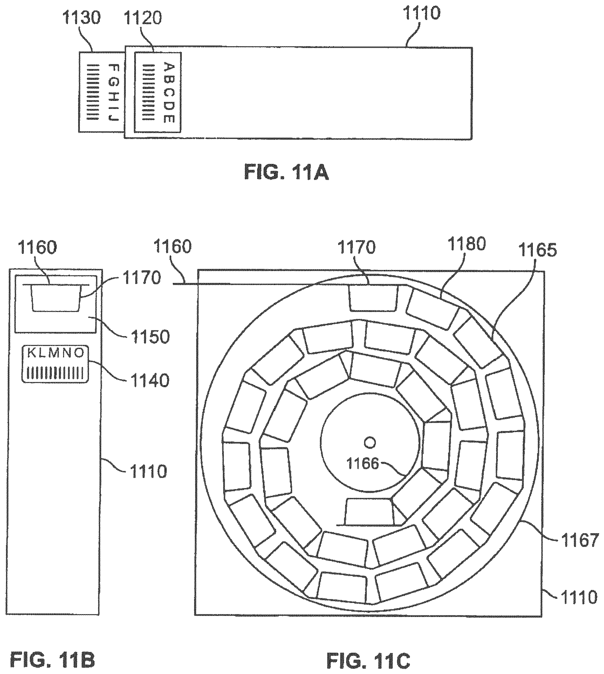

Now with reference to FIGS 11A, 11B and 11C. Depicted are multiple views of a single cassette 1110 for a home dispenser. FIG 11A is a top down view showing the single cassette 1110, which may be made of cardboard, pressboard, plastic or other suitable materials. A label 1120 is provided to show the contents of the cassette 1110. A similar label 1130 is provided on the tape.

Figure 11B is a front view of a cassette 1110 showing the leading edge of tape leader 1160 extending through opening 1150. UPD cavity 1170 is shown as it will pass through opening 1150. The label 1140 may contain information regarding the contents of 1110 in a different location that is still visible when the cassette 1110 is placed in the home dispenser.

Figure 11C is a cut-away view of cassette 1110. The cassette 1110 provides the enclosure to restrain a length of tape 1165 containing enough medication of a single type for a period of time, typically up to 31 doses for daily use for an entire month. A larger cassette could be used to contain sufficient UDP's for multiple doses per day or a longer period of time. 1110 also provides the structure and protection required for transporting, mailing, handling and dispensing the UDP's from the reel contained therein, although for confidentiality and security this cassette may be placed in an envelope or other carrier. The cassette 1110 may be refillable, recyclable or disposable.

A label 1120 is affixed to the cassette 1110 at or prior to the filling of the cassette 1110 with the ribbon 1165. The label 1120 has either or both human and machine readable information regarding the contents of the cassette 1110, including but not limited to the drug type, name, UDC, patient, time of day to be administered, quantity, physical characteristics, routing, filling and manufacturing information. In general, the label 1120 contains the information read by dispenser at the time of installation and at the time of dispensing for quality control and gathering dispensing information. The label 1130 contains information pertaining to the contents of reel 1165 and is on a leader length of tape prior to the first UDP in cassette 1110. During the prescription filling sequence, the ribbon 1165 is cut from a larger master roll. At this time it is advantageous to label the otherwise unidentified length of tape as to its origin and destination. Even though each individual UDP pocket may be labeled as to its contents (FIG. 1B, 60), additional information such as patient specific information for whom the prescription is being filled is practical to act similar to a 'router' in a manufacturing production line and as a means of identifying each individual prescription along the fulfillment path in creating a 'chain of custody' verification. This label 1130 can also have an adhesive on the back and provide a level of security and tamper resistance by ensuring that one or more doses have not been surreptitiously cut from the tape length 1165 during handling.

The tape length 1165 may be wrapped around a core 1166 with or without reel support sides 1167, or spiraled without a core, fan-folded or otherwise configured within 1110.

## Claims

1. A pharmaceutical dispensing apparatus comprising:
an enclosure (303, 500, 600, 810, 1010, 1110, 1301) having a plurality of locations (620, 817, 844, 1210), each location being assigned a location identification, each location being capable of accepting a pharmaceutical package configured to contain a plurality of serially connected pharmaceutical unit dose packages (300, 410, 715, 845, 945, 1250, 1304, 1407), the enclosure including a storage cabinet having a dispensing face (812) and an inventory loading face (814), wherein the plurality of locations is defined by conduit slots (250, 301, 520, 731, 745, 710, 844, 1210) running a length through the cabinet from the inventory loading face to the dispensing face, wherein the plurality of serially connected pharmaceutical unit dose packages are retained in the conduit slots by an arm (430, 935) under spring tension in a closed position to limit movement of a serially connected pharmaceutical unit dose packages;
a data reader (825, 940, 1306) operatively associated with said plurality of locations, wherein said reader is arranged to read data associated with said plurality of pharmaceutical packages at the time of storing the package in the dispenser and is arranged to associate said pharmaceutical package data with the location identification data indicating where the package is stored, said location identification data and pharmaceutical package data being stored in a memory;
a communications means for accepting prescription data from an associated computer, and for storing the prescription data in said memory;
a data processor associated with said memory, wherein the data processor is arranged to control the operations of the pharmaceutical dispensing apparatus, the data processor is arranged to retrieve from said memory the prescription data, location identification data and pharmaceutical package data and is arranged to provide instructions for dispensing the pharmaceuticals corresponding to the prescription data;
a dispensing mechanism (300, 400, 835, 900) wherein the dispensing mechanism is arranged to receive said instructions for dispensing and is arranged to dispense the specified quantity of unit dose pharmaceutical packages corresponding to the prescription data by moving the dispensing mechanism to the location corresponding to the assigned location identification data associated with the pharmaceutical package data stored in memory and lifting the arm retaining the serially connected pharmaceutical unit dose packages and advance the serially connected pharmaceutical unit dose packages for dispensing.

2. The pharmaceutical dispensing apparatus of Claim 1, further comprising:
a collection mechanism (855, 1315, 1316, 1410) capable of collecting in a container unit dose packages as they are dispensed from the plurality of locations;
a data labeling means for providing a label containing prescription data, said data can be read by a human or electronic reader.

3. The pharmaceutical dispensing apparatus of Claim 1 or Claim 2, wherein each plurality of locations accommodates a pharmaceutical package comprised of a tape and reel, each reel having an encoded data indicative of one or more of: the type of pharmaceutical, national drug code, strength, reel unit count, manufacturer codes, manufacturing date code, lot number and pharmaceutical dose.

4. The pharmaceutical dispensing apparatus of Claim 3, wherein the tape and reel is enclosed in a cassette or cartridge.

5. The pharmaceutical dispensing apparatus of Claim 4, wherein the cassette or cartridge incorporates a mechanism that is arranged to advance a unit dose package as the preceding unit dose package is dispensed.

6. The pharmaceutical dispensing apparatus of Claim 1 or Claim 2, wherein each plurality of locations is arranged to accept a pharmaceutical package contained within a conduit.

7. The pharmaceutical dispensing apparatus of Claim 6, wherein the conduit incorporates a mechanism that is arranged to advance a unit dose package as the preceding unit dose package is dispensed.

8. The pharmaceutical dispensing apparatus of Claim 6 or Claim 7, wherein a plurality of conduits are enclosed in a cassette or cartridge having encoded data indicative of one or more of: the type of pharmaceutical, unit count, manufacturing date code, lot number and pharmaceutical dose.

9. The pharmaceutical dispensing apparatus of any preceding Claim, wherein the associated computer is arranged to access patient medical records and to compare the patient medical records with said prescription and to determine the probability of an adverse drug reaction, and is arranged to prevent instructions for dispensing when there is a high probability of an adverse drug reaction.

10. The pharmaceutical dispensing apparatus of Claim 9, wherein a medical professional is notified when instructions for dispensing is prevented.

## Patentansprüche

1. Pharmazeutische Spendervorrichtung, umfassend:
ein Gehäuse (303, 500, 600, 810, 1010, 1110, 1301) mit einer Vielzahl von Positionen (620, 817, 844, 1210), wobei jeder Position eine Positionsidentifikation zugewiesen ist, wobei jede Position eine pharmazeutische Packung aufnehmen kann, die dazu ausgelegt ist, eine Vielzahl von seriell verbundenen pharmazeutischen Einheitsdosenpackungen (300, 410, 715, 845, 945, 1250, 1304, 1407) zu enthalten, wobei das Gehäuse einen Vorratsschrank mit einer Spenderseite (812) und einer Beladeseite (814) umfasst, wobei die Vielzahl von Positionen von Leitungsschlitzen (250, 301, 520, 731, 745, 710, 844, 1210) definiert wird, die über eine Länge von der Beladeseite bis zur Spenderseite durch den Schrank verlaufen, wobei die Vielzahl von seriell verbundenen pharmazeutischen Einheitsdosenpackungen durch einen Arm (430, 935) unter Federspannung in einer geschlossenen Position in den Leitungsschlitzen gehalten werden, um die Bewegung von seriell verbundenen pharmazeutischen Einheitsdosenpackungen zu begrenzen;
einen Datenleser (825, 940, 1306), der funktionell mit der Vielzahl von Positionen verknüpft ist, wobei der Leser dazu angeordnet ist, Daten zu lesen, die mit der Vielzahl von pharmazeutischen Packungen zu der Zeit des Speicherns der Packung in dem Spender verknüpft sind, und dazu angeordnet ist, die pharmazeutischen Packungsdaten mit den Positionsidentifikationsdaten zu verknüpfen, die anzeigen, wo die Packung gelagert ist, wobei die Positionsidentifikationsdaten und die pharmazeutischen Packungsdaten in einem Speicher gespeichert sind;
ein Kommunikationsmittel zum Annehmen von Rezeptdaten von einem verknüpften Computer und zum Speichern der Rezeptdaten in dem Speicher;
einen Datenprozessor, der mit dem Speicher verknüpft ist, wobei der Datenprozessor dazu angeordnet ist, die Vorgänge der pharmazeutischen Spendervorrichtung zu steuern, der Datenprozessor dazu angeordnet ist, die Rezeptdaten, Positionsidentifikationsdaten und pharmazeutischen Packungsdaten aus dem Speicher abzurufen, und dazu angeordnet ist, Anweisungen zum Spenden der den Rezeptdaten entsprechenden Pharmazeutika bereitzustellen;
einen Spendermechanismus (300, 400, 835, 900), wobei der Spendermechanismus dazu angeordnet ist, die Anweisungen zum Spenden zu empfangen, und dazu angeordnet ist, die angegebene Menge von den Rezeptdaten entsprechenden pharmazeutischen Einheitsdosenpackungen durch Bewegen des Spendermechanismus in die Position, die den zugewiesenen Positionsidentifikationsdaten entspricht, die mit den pharmazeutischen Packungsdaten verknüpft sind, die in dem Speicher gespeichert sind, und Anheben des Arms, der die seriell verbundenen pharmazeutischen Einheitsdosenpackungen hält und die seriell verbundenen pharmazeutischen Einheitsdosenpackungen zum Spenden vorrückt, zu spenden.

2. Pharmazeutische Spendervorrichtung nach Anspruch 1, ferner umfassend:
einen Sammelmechanismus (855, 1315, 1316, 1410), der Einheitsdosenpackungen in einem Behälter sammeln kann, wenn sie aus der Vielzahl von Positionen ausgegeben werden;
ein Datenetikettiermittel zur Bereitstellung eines Etiketts, das Rezeptdaten enthält, die Daten können von einem menschlichen oder elektronischen Leser gelesen werden.

3. Pharmazeutische Spendervorrichtung nach Anspruch 1 oder Anspruch 2, wobei jede Vielzahl von Positionen eine pharmazeutische Packung beherbergt, die aus einer Bandspule besteht, wobei jede Spule kodierte Daten aufweist, die eins oder mehrere anzeigen von: der Art des Pharmazeutikums, nationalem Arzneimittelcode, Stärke, Spuleneinheitenanzahl, Herstellercodes, Herstellerdatumscode, Chargennummer und pharmazeutischer Dosis.

4. Pharmazeutische Spendervorrichtung nach Anspruch 3, wobei die Bandspule in einer Kassette oder Kartusche enthalten ist.

5. Pharmazeutische Spendervorrichtung nach Anspruch 4, wobei die Kassette oder Kartusche einen Mechanismus aufweist, der dazu angeordnet ist, eine Einheitsdosenpackung vorzurücken, wenn die vorhergehenden Einheitsdosenpackung ausgegeben wird.

6. Pharmazeutische Spendervorrichtung nach Anspruch 1 oder Anspruch 2, wobei jede Vielzahl von Positionen dazu angeordnet ist, eine in einem Kanal enthaltene pharmazeutische Packung aufzunehmen.

7. Pharmazeutische Spendervorrichtung nach Anspruch 6, wobei der Kanal einen Mechanismus aufweist, der dazu angeordnet ist, eine Einheitsdosenpackung vorzurücken, wenn die vorhergehende Einheitsdosenpackung ausgegeben wird.

8. Pharmazeutische Spendervorrichtung nach Anspruch 6 oder Anspruch 7, wobei eine Vielzahl von Kanälen in einer Kassette oder Kartusche enthalten sind, die kodierte Daten aufweist, die eins oder mehrere anzeigen von: der Art des Pharmazeutikums, Einheitenanzahl Herstellungsdatumscode, Chargennummer und pharmazeutischer Dosis.

9. Pharmazeutische Spendervorrichtung nach einem vorhergehenden Anspruch, wobei der verknüpfte Computer dazu angeordnet ist, auf die Patientenakten zuzugreifen und die Patientenakten mit dem Rezept zu vergleichen und die Wahrscheinlichkeit einer Arzneimittelnebenwirkung zu bestimmen, und dazu angeordnet ist, Anweisungen zum Spenden zu verhindern, wenn die Wahrscheinlichkeit einer Arzneimittelnebenwirkung hoch ist.

10. Pharmazeutische Spendervorrichtung nach Anspruch 9, wobei eine medizinische Fachkraft in Kenntnis gesetzt wird, wenn Anweisungen zum Spenden verhindert werden.

## Revendications

1. Appareil de distribution de produit pharmaceutique comprenant :
une enceinte (303, 500, 600, 810, 1010, 1110, 1301) comportant une pluralité d'emplacements (620, 817, 844, 1210), chaque emplacement recevant une identification d'emplacement, chaque emplacement étant susceptible d'accepter une boîte de produit pharmaceutique conçue pour contenir une pluralité de boîtes de doses unitaires de produit pharmaceutique reliées en série (300, 410, 715, 845, 945, 1250, 1304, 1407), l'enceinte comprenant une armoire de stockage possédant une face de distribution (812) et une face de chargement de stock (814), dans laquelle la pluralité d'emplacements est définie par des fentes de conduit (250, 301, 520, 731, 745, 710, 844, 1210) parcourant une longueur à travers l'armoire depuis la face de chargement de stock vers la face de distribution, dans laquelle la pluralité de boîtes de doses unitaires de produit pharmaceutique reliées en série est retenue dans les fentes de conduit par un bras (430, 935) sous une tension de ressort dans une position fermée afin de limiter un mouvement de boîtes de doses unitaires de produit pharmaceutique reliées en série ;
un lecteur de données (825, 940, 1306) associé fonctionnellement à ladite pluralité d'emplacements, ledit lecteur étant conçu pour lire des données associées à ladite pluralité de boîtes de produit pharmaceutique au moment du stockage de la boîte dans le distributeur et étant conçu pour associer lesdites données de boîtes de produit pharmaceutique aux données d'identification d'emplacement indiquant où est stockée la boîte, lesdites données d'identification d'emplacement et lesdites données de boîtes de produit pharmaceutique étant mémorisées dans une mémoire ;
un moyen de communication pour accepter des données d'ordonnance provenant d'un ordinateur associé, et pour mémoriser les données d'ordonnance dans ladite mémoire ;
un processeur de données associé à ladite mémoire, le processeur de données étant conçu pour commander les opérations de l'appareil de distribution de produit pharmaceutique, le processeur de données étant conçu pour récupérer les données d'ordonnance, les données d'identification d'emplacement et les données de boîtes de produit pharmaceutique dans ladite mémoire et étant conçu pour fournir des instructions pour distribuer les produits pharmaceutiques correspondant aux données d'ordonnance ;
un mécanisme de distribution (300, 400, 835, 900), le mécanisme de distribution étant conçu pour recevoir lesdites instructions de distribution et étant conçu pour distribuer la quantité spécifiée de boîtes de doses unitaires de produit pharmaceutique correspondant aux données d'ordonnance en déplaçant le mécanisme de distribution vers l'emplacement correspondant aux données d'identification d'emplacement attribué associées aux données de boîtes de produit pharmaceutique mémorisées dans la mémoire et en levant le bras retenant les boîtes de doses unitaires de produit pharmaceutique reliées en série et pour faire avancer les boîtes de doses unitaires de produit pharmaceutique reliées en série pour les distribuer.

2. Appareil de distribution de produit pharmaceutique selon la revendication 1, comprenant en outre :
un mécanisme de collecte (855, 1315, 1316, 1410) susceptible de collecter dans une unité de contenant des boîtes de doses à mesure qu'elles sont distribuées depuis la pluralité d'emplacements ;
un moyen d'étiquetage de données pour fournir une étiquette contenant des données d'ordonnance, lesdites données pouvant être lues par un humain ou un lecteur électronique.

3. Appareil de distribution de produit pharmaceutique selon la revendication 1 ou la revendication 2, dans lequel chaque emplacement de la pluralité d'emplacements accueille une boîte de produits pharmaceutiques composée d'une bande et d'un rouleau, chaque rouleau ayant des données codées faisant état d'un ou plusieurs parmi : le type de produit pharmaceutique, le code national du médicament, la concentration, le nombre d'unités de rouleaux, les codes de fabricant, le code de date de fabrication, le numéro de lot et la dose de produit pharmaceutique.

4. Appareil de distribution de produit pharmaceutique selon la revendication 3, dans lequel la bande et le rouleau sont enfermés dans une cassette ou une cartouche.

5. Appareil de distribution de produit pharmaceutique selon la revendication 4, dans lequel la cassette ou la cartouche intègre un mécanisme qui est conçu pour faire avancer une boîte de doses unitaires lorsque la boîte de doses unitaires précédente est distribuée.

6. Appareil de distribution de produit pharmaceutique selon la revendication 1 ou la revendication 2, dans lequel chaque emplacement de la pluralité d'emplacements est conçu pour accepter une boîte de produit pharmaceutique contenue dans un conduit.

7. Appareil de distribution de produit pharmaceutique selon la revendication 6, dans lequel le conduit intègre un mécanisme qui est conçu pour faire avancer une boîte de doses unitaires lorsque la boîte de doses unitaires précédente est distribuée.

8. Appareil de distribution de produit pharmaceutique selon la revendication 6 ou la revendication 7, dans lequel de multiples conduits sont enfermés dans une cassette ou une cartouche ayant des données codées faisant état d'un ou plusieurs parmi : le type de produit pharmaceutique, le nombre d'unités, le code de date de fabrication, le numéro de lot et la dose de produit pharmaceutique.

9. Appareil de distribution de produit pharmaceutique selon l'une quelconque des revendications précédentes, dans lequel l'ordinateur associé est conçu pour accéder à des dossiers médicaux de patients et pour comparer les dossiers médicaux de patients avec ladite ordonnance et pour déterminer l'éventualité d'une réaction médicamenteuse indésirable, et est conçu pour empêcher que les instructions réalisent une distribution lorsqu'il existe un risque élevé de réaction médicamenteuse indésirable.

10. Appareil de distribution de produit pharmaceutique selon la revendication 9, dans lequel un professionnel médical est notifié lorsque des instructions de distribution sont empêchées.
